(19) Europäisches Patentamt
European Patent Office
Office européen des brevets

(11) EP 1 342 466 B1

(12) **FASCICULE DE BREVET EUROPEEN**

(45) Date de publication et mention de la délivrance du brevet:
**17.10.2007 Bulletin 2007/42**

(51) Int Cl.:
***A61K 8/66*** *(2006.01)* ***A61K 8/49*** *(2006.01)*
***A61Q 5/04*** *(2006.01)*

(21) Numéro de dépôt: **03290466.6**

(22) Date de dépôt: **27.02.2003**

(54) **Composition non colorante contenant un précurseur et un catalyseur de réaction d'oxydation**

Nichtverfärbende Zusammensetzung enthaltend einen PRECURSOR und einen Katalysator für Oxidationsreaktionen

Non-colouring composition containing a precursor and a catalyst for oxidative reaction

(84) Etats contractants désignés:
**AT BE BG CH CY CZ DE DK EE ES FI FR GB GR HU IE IT LI LU MC NL PT SE SI SK TR**

(30) Priorité: **01.03.2002 FR 0202652**

(43) Date de publication de la demande:
**10.09.2003 Bulletin 2003/37**

(73) Titulaire: **L'ORÉAL**
**75008 Paris (FR)**

(72) Inventeur: **Plos, Grégory**
**75011 Paris (FR)**

(74) Mandataire: **Casalonga, Axel**
**Bureau Casalonga & Josse**
**Bayerstrasse 71/73**
**80335 München (DE)**

(56) Documents cités:
**EP-A- 1 147 763**
**EP-A- 1 157 684**
**WO-A-97/19999**
**US-A- 5 961 667**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente demande se rapporte à une composition cosmétique non colorante destinée à la mise en forme durable des matières kératiniques ou à leur texturisation, ainsi qu'à un procédé mettant en oeuvre une telle composition.

De nombreuses personnes ont des difficultés à donner à leur chevelure la forme qu'elles désirent. C'est en particulier le cas des personnes ayant une chevelure de cheveux fins. Les cheveux fins n'ont pas suffisamment de rigidité pour qu'une fois la coiffure mise en place, cette dernière puisse résister dans le temps. La coiffure, soumise aux mouvements ainsi qu'à l'humidité et aux intempéries, perd sa forme assez rapidement. Ce phénomène est spécialement accentué dans le cas des chevelures de cheveux fins, mais est aussi un problème dans le cas des chevelures de cheveux moyens et épais.

On recherche depuis longtemps des traitements permettant aux coiffures de mieux résister dans le temps.

Il existe des produits basés sur l'emploi de polymères.

Ces produits sont présentés sous forme de laques, de sprays, de gels et de mousses. Ces laques et les sprays ont un effet puissant, mais donnent à la coiffure un aspect et un toucher artificiels du fait que l'on crée des soudures entre les cheveux.

Les gels et les mousses, lorsqu'ils sont employés en combinaison avec un brushing, amènent une meilleure résistance de la forme tout en laissant un aspect et un toucher assez naturels. Cependant, l'effet est assez modéré et dans de nombreux cas, en particulier pour les cheveux fins, est considéré comme insuffisant.

Une autre technique est basée sur la modification des liaisons disulfures des cheveux. Les produits dits de "permanente" employés à cet effet contiennent généralement des thiols ou des sulfites, capables de "réduire" les ponts disulfures de la kératine qui est le composant essentiel des cheveux et ainsi "fragiliser" la structure des cheveux. Pendant ce temps, étant soumis à une contrainte mécanique (ils sont par exemple enroulés), les cheveux prennent une nouvelle forme. A la fin du traitement, les ponts disulfures du cheveu étant réduits, on doit les réoxyder pour rétablir la cohésion des cheveux. Ce procédé entraîne des dégradations des fibres kératiniques. Par ailleurs, la sensibilité aux colorations et décolorations ultérieures des fibres ainsi traitées est modifiée. Un autre inconvénient est que le procédé est assez long car il exige plusieurs étapes : enroulage, pose d'un premier produit, pause, rinçage, pose d'un second produit, pause, rinçage, déroulage, rinçage et lavage terminal. En outre, ces procédés sont assez inconfortables, notamment à cause de l'odeur du premier liquide, de l'odeur dégagée pendant la première pause et de l'odeur résiduelle laissée dans les cheveux.

Cependant, ces procédés sont avantageux dans la mesure où leur effet est durable et résiste aux shampooings.

De manière avantageuse et inattendue, la Demanderesse a trouvé une composition qui ne présente pas les inconvénients des compositions de l'art antérieur, tout en conduisant à une mise en forme durable des fibres kératiniques et en particulier des cheveux.

En effet, cette composition comprend un agent susceptible de polymériser par oxydation à l'intérieur de la fibre. Par conséquent, l'utilisation de ces compositions ne confère pas à la coiffure un aspect et un toucher artificiels.

Ces compositions permettent une application en une étape de durée nettement inférieure à la durée de mise en oeuvre d'une permanente classique.

De plus, les compositions selon l'invention présentent les avantages de conférer après lavage et séchage, un effet de corps notable c'est-à-dire un effet qui se traduit par la sensation, au toucher, que les cheveux sont plus épais. Cette notion est spécialement perceptible sur des cheveux fins. On parle alors de texturisation des cheveux.

A cause des produits utilisés et de la durée d'application, les cheveux subissent une altération moindre que lorsqu'ils sont traités par un procédé de permanente classique.

L'invention permet d'obtenir sur les cheveux même fins un effet coiffant notable, une tenue dans le temps améliorée avec en particulier une résistance à plusieurs shampooings et un effet de brillance qu'il est difficile d'obtenir avec les produits du commerce.

Ainsi, un objet de la présente demande concerne une composition cosmétique non colorante, cette composition contenant, dans un milieu cosmétiquement acceptable, au moins un composé choisi parmi la 1(isobutyloxycarbonyle) 5,6-dihydroxyindoline et le 1-hydroxyéthyl 4-hydroxyindole en tant qu'agent susceptible de polymériser par oxydation et au moins une laccase comme agent catalyseur de cette réaction de polymérisation par oxydation.

Par composition non colorante, on entend selon la présente invention, une composition qui, appliquée sur les cheveux, entraîne une modification du facteur colorimétrique "ΔE" des cheveux après traitement inférieure à 5 unités et de préférence inférieure à 2 unités.

Le facteur colorimétrique "ΔE" est défini par la méthode dite L*a*b*, encore appelée "méthode CIELAB" de la manière suivante :

$$\Delta E = [(L^* \text{ final} - L^* \text{ initial})^2 + (a^* \text{ final} - a^* \text{ initial})^2 + (b^* \text{ final} - b^* \text{ initial})^2]^{1/2}$$

dans laquelle L* représente la luminance, a* la couleur sur l'axe vert-rouge et b* représente la couleur sur l'axe bleu-jaune.

Ce facteur colorimétrique se mesure à l'aide d'un colorimètre tel que le colorimètre MINOLTA CM2002. L'erreur sur la mesure "ΔE" est très faible.

Les compositions selon l'invention contiennent de préférence de 0,001% à 20% et de manière encore plus préférée de 0,05% à 10% en poids d'agent(s) susceptible(s) de polymériser par oxydation par rapport au poids total de ladite composition.

Par "milieu cosmétiquement acceptable" au sens de la présente demande, on entend un milieu aqueux ou un milieu contenant un ou plusieurs solvants ou encore un milieu constitué par un mélange d'eau et d'un ou plusieurs solvants.

Les solvants peuvent être en particulier choisis parmi les monoalcools et de préférence les alcools inférieurs tel que l'éthanol, les polyols tels que le propylène glycol, la glycérine, les polyéthylène glycols, les éthers ou les esters de ces monoalcools ou polyols.

La polymérisation par oxydation est de préférence effectuée en utilisant l'oxygène de l'air en tant qu'agent oxydant.

Les compositions selon l'invention contiennent de préférence de 0,00001% à 5% et de manière encore plus préférée de 0,001% à 1% en poids d'agent(s) catalyseur(s) d'oxydation par rapport au poids total de ladite composition.

Selon une variante particulière de l'invention, la composition cosmétique comprend en outre au moins un médiateur de la réaction d'oxydation. Ces médiateurs sont destinés à faciliter la réaction d'oxydation.

Une forme particulière de l'invention consiste à employer i) un indole susceptible de polymériser par oxydation, ii) un catalyseur d'oxydation et iii) un médiateur d'oxydation.

Le ou les médiateurs enzymatiques utilisés dans la composition conforme à l'invention permettent d'augmenter l'activité enzymatique de l'oxydoréductase utilisée et peuvent par exemple être choisis parmi les composés de formule (I) suivante, et leurs possibles formes tautomères

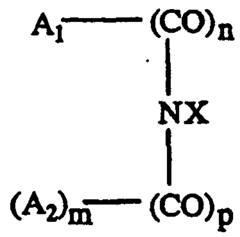

(I)

dans laquelle :

- $A_1$ et $A_2$, identiques ou différents, représentent :

  a) un radical aliphatique saturé ou insaturé, linéaire ou ramifié, comportant de 1 à 30 atomes de carbone, ledit radical aliphatique pouvant ou non être substitué par un ou plusieurs radicaux hydroxyle, halogène, sulfo, carboxy, nitro ou phényle ;
  b) un radical hétérocyclique comprenant de 1 à 4 hétéroatomes et de 5 à 10 chaînons pouvant ou non être substitué par un ou plusieurs radicaux alkyles en $C_1$-$C_4$, halogène, phényle, hydroxyle, ou aralkyle en $C_7$-$C_{10}$ ;
  c) un radical aromatique comprenant de 6 à 10 chaînons, ledit radical aromatique pouvant ou non être substitué par un ou plusieurs radicaux alkyles en $C_1$-$C_4$, halogène, sulfo, carboxy, nitro, hydroxyle, ou nitroso ;

l'atome d'azote du groupement NX pouvant former avec les groupements $A_1$-(CO)n et $A_2$-(CO)p un hétérocycle comprenant de 5 à 18 chaînons, ledit hétérocycle pouvant ou non être substitué par un ou plusieurs radicaux alkyles en $C_1$-$C_4$, hydroxyle, phényle, halogène, sulfo, carboxy ou nitro ;

- X représente un groupement -OH, =O, =S, →O ou →S ;
- m, n et p, indépendamment les uns des autres, sont des nombres entiers égaux à 0 ou 1.

Parmi les médiateurs enzymatiques de formule (I) ci-dessus, on peut notamment citer l'hydroxylamine, la N, N-dipropyl hydroxylamine, la N,N-diisopropyl hydroxylamine, la phényl hydroxylamine, la N-acétyl hydroxylamine, le 1-phényl-1H-1,2,3-triazole-1-oxyde, le 2,4,5-triphényl-2H-1,2,3-triazol-1-oxyde, le 1-hydroxy benzotriazole, l'acide 1-hydroxy benzotriazole sulfonique, le 1-hydroxy benzimidazole, le N-hydroxy phtalimide, le N-hydroxy succinimide, la quinoline N-oxyde, l'isoquinoline N-oxyde, la 1-hydroxy pipéridine, l'acide violurique, la 4-hydroxy 3-nitroso coumarine, l'acide 1,3-diméthyl 5-nitroso barbiturique, le 1-nitroso 2-naphtol, l'acide 2-nitroso 1-naphtol 4-sùlfonique, le 2-nitroso 1-naphtol, l'acide 1-nitroso 2-naphtol 3,6-disulfonique, et le 2,4-dinitroso 1,3-dihydroxy benzène.

Le ou les médiateurs enzymatiques utilisés dans la composition prête à l'emploi conforme à l'invention peuvent également être choisis parmi l'acide syringique et ses esters ; l'acétosyringone ; le syringaldéhyde ; l'acide parahydroxycinnamique ; la vanilline ; la 7-hydroxycoumarine ; le 2,4-dichloro phénol ; le parahydroxybenzènesulfonate ; le 2,2'-azino-bis-(3-éthylbenzothiazoline-6-sulfonate) ; les phénothiazines telles que la 10-méthylphénothiazine ; les benzidines telles que la 3,3'-diméthyl benzidine ; les dérivés aminés de l'acide 2-naphtalène sulfonique ; la L-tyrosine ; l'acide férulique ; l'acide caféique, l'acide chlorogénique, et l'acide sinapique.

Les compositions selon l'invention contiennent de préférence de 0,00001% à 5% et de manière encore plus préférée de 0,001% à 1% en poids de médiateur par rapport au poids total de ladite composition.

La composition cosmétique selon la présente invention peut encore contenir un autre agent choisi dans le groupe formé par les agents tensioactifs non ioniques, anioniques, cationiques ou amphotères, les agents épaississants organiques ou minéraux, les polymères non ioniques, anioniques, cationiques ou amphotères, coiffants ou non, les silicones volatiles ou non, linéaires ou cycliques , organomodifiées ou non, les vitamines ou provitamines, les parfums.

Un second objet de l'invention réside dans l'utilisation des compositions non colorantes selon l'invention définies ci-dessus pour la mise en forme durable des matières kératiniques et en particulier des fibres kératiniques humaines tels que les cheveux.

Un troisième objet de l'invention réside dans l'utilisation des compositions non colorantes selon l'invention définies ci-dessus pour la texturisation des matières kératiniques et en particulier des fibres kératiniques humaines tels que les cheveux.

La présente invention a encore pour quatrième objet un procédé de traitement des matières kératiniques et en particulier, des cheveux tel que l'on applique sur ces matières kératiniques, une composition cosmétique selon la présente invention.

Ce procédé selon la présente invention peut être mis en oeuvre sur des cheveux secs ou humides. De préférence, on privilégiera une application sur cheveux humides.

La durée d'application de la composition selon l'invention est comprise entre 10 secondes et 1 heure, de préférence cette application durera entre 10 secondes et 30 minutes.

De préférence, après application de la composition, les matières kératiniques sont rincées.

Le procédé de traitement des matières kératiniques selon la présente invention, peut en outre être combiné à un ou plusieurs (2, 3, 4) autres traitements classiques.

Ainsi, le traitement selon la présente invention, peut être appliqué avant, pendant ou après ces autres traitements.

Ainsi, on peut mettre en oeuvre le procédé de mise en forme permanente selon la présente invention, avant ou pendant ou après un traitement de coloration, de décoloration ou encore de permanente classique.

Dans le cas de traitement en plusieurs étapes, le traitement selon la présente invention peut avoir lieu entre deux étapes dudit traitement classique.

Les exemples qui suivent illustrent l'invention sans en limiter la portée.

## Exemple 1

On réalise la composition suivante :

| | |
|---|---|
| 1(isobutyloxycarbonyl) 5,6 dihydroxy indoline (structure ci-dessous) | $3.10^{-3}$ mole |
| LACCASE | 0.015g en protéines soit 400 000 unités |
| Tampon phosphate pH7/éthanol (50/50) | qs 100g |

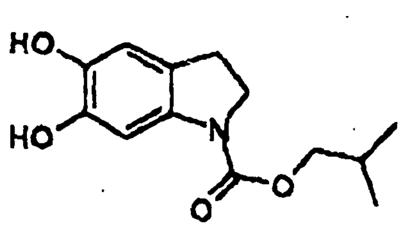

L'activité en unités de laccase est, à un pH donné (ici 1), la quantité d'enzymes qui provoque à 25°C une modification d'absorbance de 0.001 à 435 nm à partir d'une solution 27mM de paraphénylènediamine pour un trajet optique de 1cm.

La composition est appliquée sur cheveux naturels blancs. Après 30 minutes de pose, on rince. On note que les cheveux restent pratiquement blancs (facteur colorimétrique ΔE de 3,76 points).

En comparaison avec des cheveux non traités, les cheveux présentent un effet de corps notable (texturisation). Cet effet de corps est retrouvé après lavage puis séchage des cheveux.

**Exemple 2)**

On réalise la composition suivante :

| | |
|---|---|
| 1(isobuxyloxycarbonyl) 5,6 dihydroxy indoline (structure ci-dessous) | $3.10^{-3}$ mole |
| LACCASE | 0,015g en protéines soit 400 000 unités |
| Acide violurique | 0,5g |
| Tampon phosphate pH7/éthanol (50/50) | qs 100g |

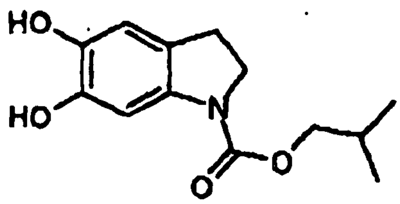

La composition est appliquée sur cheveux naturels blancs. Après 30 minutes de pose, on rince. On note que les cheveux restent blancs (facteur colorimétrique ΔE de 1,34 points).

En comparaison avec des cheveux non traités, les cheveux présentent un effet de corps marqué. Cet effet de corps est retrouvé après lavage puis séchage des cheveux.

**Exemple 3)**

On réalise la composition suivants :

| | |
|---|---|
| 1-hydroxyéthyl 4-hydroxyindole (structure donnée ci-après) | 0,01g |
| LACCASE | 0,015g en protéines soit 400 000 unités |
| Tampon phosphate pH7/éthanol (70/30) | qs 100g |

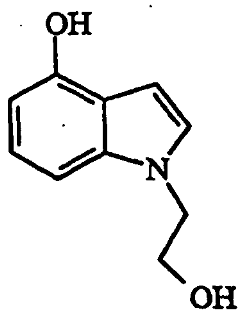

La composition est appliquée sur cheveux naturels blancs. Après 30 minutes de pose, on rince. On note que les cheveux restent blancs (facteur colorimétrique ΔE de 1,9 points).

En comparaison avec des cheveux non traités, les cheveux présentent un effet de corps notable. Cet effet de corps est retrouvé après lavage puis séchage des cheveux.

## Revendications

1. Composition cosmétique non colorante contenant, dans un milieu cosmétiquement acceptable, au moins un composé choisi parmi la 1(isobutyloxycarbonyl) 5,6 dihydroxy-indoline et le 1-hydroxyéthyl 4-hydroxyindole en tant qu'agent susceptible de polymériser par oxydation et au moins une laccase en tant qu'agent catalyseur de cette réaction de polymérisation par oxydation.

2. Composition cosmétique selon l'une des revendications précédentes telle qu'elle contient de préférence de 0,00 1 % à 20 % et de manière encore plus préférée de 0,05% à 10% en poids d'agent(s) susceptible(s) de polymériser par oxydation par rapport au poids total de ladite composition.

3. Composition cosmétique selon l'une des revendications précédentes telle qu'elle contient de préférence de 0,00001% à 5% et de manière encore plus préférée de 0,001% à 1% en poids d'agent(s) catalyseur(s) d'oxydation par rapport au poids total de ladite composition.

4. Composition cosmétique selon l'une des revendications précédentes telle qu'elle comprend au moins un médiateur de la réaction d'oxydation.

5. Composition cosmétique selon l'une des revendications précédentes telle qu'elle comprend au moins un autre agent choisi dans le groupe formé par les agents tensioactifs non ioniques, anioniques, cationiques ou amphotères, les agents épaississants organiques ou minéraux, les polymères non ioniques, anioniques, cationiques ou amphotères, coiffants ou non, les silicones volatiles ou non, linéaires ou cycliques , organomodifiées ou non, les vitamines ou provitamines, les parfums.

6. Procédé de traitement des matières kératiniques **caractérisé en ce que** l'on applique sur ces matières kératiniques une composition cosmétique selon l'une des revendications 1 à 5 pendant une durée comprise entre 10 secondes et une heure, de préférence entre 10 secondes et 30 minutes.

7. Procédé de traitement selon la revendication 6 tel que l'application de la composition cosmétique est suivie d'une étape de rinçage.

8. Procédé de traitement selon la revendication 6 à 7 tel que ce procédé est combiné avec au moins un autre traitement choisi parmi les colorations, décolorations, permanentes classiques.

9. Utilisation d'une composition selon l'une des revendications 1 à 5 pour la mise en forme durable dos matières kératiniques et en particulier des fibres kératiniques humaines tels que les cheveux.

10. Utilisation d'une composition selon l'une des revendications 1 à 5 pour la texturisation des matières kératiniques et en particulier des fibres kératiniques humaines tels que les cheveux.

## Claims

1. Non-colouring cosmetic composition containing, in a cosmetically acceptable medium, at least one compound chosen from 1-(isobutyloxycarbonyl)-5,6-dihydroxyindoline and 1-hydroxyethyl-4-hydroxyindole as an agent capable of undergoing oxidative polymerization, and at least one laccase as catalyst for this oxidative polymerization reaction.

2. Cosmetic composition according to the preceding claim, such that it preferably contains from 0.001% to 20% and even more preferably from 0.05% to 10% by weight of agent(s) capable of undergoing oxidative polymerization, relative to the total weight of the said composition.

3. Cosmetic composition according to either of the preceding claims, such that it preferably contains from 0.00001%

to 5% and even more preferably from 0.001% to 1% by weight of oxidation catalyst(s) relative to the total weight of the said composition.

**4.** Cosmetic composition according to one of the preceding claims, such that it comprises at least one oxidation reaction mediator.

**5.** Cosmetic composition according to one of the preceding claims, such that it comprises at least one other agent chosen from the group formed by nonionic, anionic, cationic or amphoteric surfactants, organic or mineral thickeners, styling or non-styling nonionic, anionic, cationic or amphoteric polymers, volatile or non-volatile, linear or cyclic, organomodified or non-organomodified silicones, vitamins or provitamins, and fragrances.

**6.** Process for treating keratin materials, **characterized in that** a cosmetic composition according to one of Claims 1 to 5 is applied to these keratin materials for a time of between 10 seconds and one hour and preferably between 10 seconds and 30 minutes.

**7.** Treatment process according to Claim 6, such that the application of the cosmetic composition is followed by a rinsing step.

**8.** Treatment process according to Claim 6 or 7, such that this process is combined with at least one other treatment chosen from standard dyeing, bleaching or permanent-waving.

**9.** Use of a composition according to any one of Claims 1 to 5, for durably shaping keratin materials and in particular human keratin fibres such as the hair.

**10.** Use of a composition according to one of Claims 1 to 5, for texturizing keratin materials and in particular human keratin fibres such as the hair.

**Patentansprüche**

**1.** Nichtfärbende kosmetische Zusammensetzung, die in einem kosmetisch akzeptablen Medium als Stoff, der befähigt ist, durch Oxidation zu polymerisieren, mindestens eine Verbindung, die unter 1-(isobutyloxycarbonyl)5,6-Dihydroxyindolin und 1-Hydroxyethyl-4-hydroxyindol ausgewählt ist, und mindestens eine Laccase als Katalysator für eine solche oxidative Polymerisationsreaktion enthält.

**2.** Kosmetische Zusammensetzung nach einem der vorhergehenden Ansprüche, die vorzugsweise 0,001 bis 20 Gew.-% und noch bevorzugter 0,05 bis 10 Gew.-% des Stoffes oder der Stoffe, der (die) befähigt ist (sind), durch Oxidation zu polymerisieren, bezogen auf das Gesamtgewicht der Zusammensetzung, enthält.

**3.** Kosmetische Zusammensetzung nach einem der vorhergehenden Ansprüche, die vorzugsweise 0,00001 bis 5 Gew.-% und noch bevorzugter 0,001 bis 1 Gew.-% des Oxidationskatalysators oder der Oxidationskatalysatoren, bezogen auf das Gesamtgewicht der Zusammensetzung, enthält.

**4.** Kosmetische Zusammensetzung nach einem der vorhergehenden Ansprüche, die mindestens einen Mediator der Oxidationsreaktion enthält.

**5.** Kosmetische Zusammensetzung nach einem der vorhergehenden Ansprüche, die mindestens einen weiteren Stoff enthält, der unter den nichtionischen, anionischen, kationischen oder amphoteren grenzflächenaktiven Stoffen, organischen oder anorganischen Verdickungsmitteln, nichtionischen, anionischen, kationischen oder amphoteren Polymeren, die frisurgebende Eigenschaften haben oder nicht, flüchtigen oder nichtflüchtigen, linearen oder cyclischen, organomodifizierten oder nicht organomodifizierten Siliconen, Vitaminen oder Provitaminen und Parfums ausgewählt ist.

**6.** Verfahren zur Behandlung von Keratinsubstanzen, **dadurch gekennzeichnet, dass** auf die Keratinsubstanzen eine kosmetische Zusammensetzung nach einem der Ansprüche 1 bis 5 während einer Zeitspanne von 10 Sekunden bis eine Stunde und vorzugsweise 10 Sekunden bis 30 Minuten aufgebracht wird.

**7.** Verfahren zur Behandlung nach Anspruch 6, wobei nach dem Aufbringen der kosmetischen Zusammensetzung ein

Spülschritt folgt.

8. Verfahren zur Behandlung nach Anspruch 6 oder 7, wobei dieses Verfahren mit mindestens einer weiteren Behandlung kombiniert wird, die unter den herkömmlichen Färbungen, Entfärbungen und permanenten Verformungen ausgewählt ist.

9. Verwendung einer Zusammensetzung nach einem der Ansprüche 1 bis 5 zur nachhaltigen Formgebung von Keratinsubstanzen und insbesondere menschlichen Keratinfasern, wie Haaren.

10. Verwendung einer Zusammensetzung nach einem der Ansprüche 1 bis 5 zur Texturierung von Keratinsubstanzen und insbesondere menschlichen Keratinfasern wie Haaren.